# EUROPEAN PATENT APPLICATION

(11) **EP 2 168 527 A1**
(43) Date of publication of application: **31.03.2010**
(21) Application number: 07788583.8
(22) Date of filing: 07.06.2007
(51) Int. Cl.: A61C 8/00, A61L 27/06

(54) **NOVEL SURFACE OF METAL IMPLANTS BASED ON TITANIUM, TO BE INSERTED INTO OSSEOUS TISSUE**

(30) Priority: 01.06.2007 ES 200701518
(71) Applicant: Garcia Saban, Francisco Javier, 08211 Castellar del Valles, Barcelona (ES); Garcia Saban, Juan Carlos, 08211 Castellar del Valles, Barcelona (ES); Garcia Saban, Miguel Angel, 08211 Castellar del Valles, Barcelona (ES)
(72) Inventor: Garcia Saban, Francisco Javier, 08211 Castellar del Valles, Barcelona (ES); Garcia Saban, Juan Carlos, 08211 Castellar del Valles, Barcelona (ES); Garcia Saban, Miguel Angel, 08211 Castellar del Valles, Barcelona (ES)
(74) Representative: Carpintero Lopez, Francisco
(86) International application number: PCT/ES2007/000335
(87) International publication number: WO 2008/145768

(57) **Abstract**

The invention defines a surface of a titanium-based metal implant to be inserted into bone tissue, **characterised in that** it comprises substantially pure titanium oxide and has a thickness of 8-50nm. Also, the invention defines a procedure for obtaining the surface, as well as the metal implant that exhibits it. This surface exhibits good micrometric roughness and a composition that is practically free from impurities and a thickness that is approximately three times the thickness of conventional surfaces; these characteristics provide it with very good osseointegration properties.

## Description

### FIELD OF THE INVENTION

The invention refers to the field of metal implants to be inserted into bone tissue. Specifically, the invention refers to a metal implant surface that exhibits good roughness and has an optimised chemical composition and thickness resulting in a better cellular response, and therefore, a better implant-bone binding. The invention also refers to a procedure for obtaining this surface as well as the metal implant that exhibits it.

### BACKGROUND OF THE INVENTION

As is well known in the state of the art, some metals or metal alloys such as titanium, zirconium, hafnium, tantalum, niobium or alloys of these are used to form relatively strong links with bone tissue. In particular, metal implants of titanium and its alloys have been known since approximately 1950 for their properties of binding well to bone tissue. This binding was called osseointegration by Branemark et al. (Branemark et al., "Osseointegrated implants in the treatment of the edentulous jaw. Experience from a 10-year period", Scand. J. Plast. Reconstr. Surg., II, suppl 16 (1977)).

Although the binding between this metal and bone tissue is relatively strong, it is desirable to improve this binding. There are many methods developed in the state of the art to treat such metal implants to obtain a suitable surface on them to improve their osseointegration. The term "surface" is understood to refer to the superficial layer or most external zone of an implant, composed mainly of the oxide of the corresponding metal, the physical properties of which are clearly different from the massive material that the implant is made of.

Some of these methods are directed to altering the morphology of this superficial layer, increasing its roughness, in order to provide a higher area of contact, and therefore of binding, between the implant and the bone tissue, resulting in higher mechanical retention and strength, that is, in a better osseointegration of the implant.

The reason behind these procedures for increasing surface roughness are the studies carried out in the last few years (Buser et al., "Influence of surface characteristics on bone integration of titanium implants. A histomorphometric study in miniature pigs", J Biom Mater Res, (1991), 25:889-902; Wennerberg et al., "Torque and histomorphometric evaluation of c.p. titanium screws blasted with 25- and 75-um-sized particles of Al2O3", J Biom Mater Res, (1996); 30:251-260; Buser et al., "Removal torque value of titanium implants in the maxilla of miniature pigs", J Oral Maxillofac Implants (1998) 13:611-619; and Lazzara et al., "Bone response to dual acid-etched and machined titanium implant surfaces", Bone Engineering, cap.34 (2000) J.E.Davies eds.), which demonstrate that osseointegration of the implant in the short and medium term is improved by a micrometric surface roughness.

Also, other studies (Buser et al. 1991, supra; Cochran et al., "Attachment and growth of periodontal cells on smooth and rough titanium", Int. J Oral Maxillofac Implants (1994) 9:289-297; Martin et al., "Effect of titanium surface roughness on proliferation, differentiation, and protein synthesis of human osteoblast-like cells (MG63)", J Biom Mat Res (1995) 29:389-401; Lazzara et al. 2000, supra; and Orsini et al., "Surface analysis of machined vs sandblasted and acid-etched titanium implants", J. Oral Maxillofac Implants (2000) 15:779-784) have demonstrated that the existence of a superficial layer on the implant of a micrometric roughness improves osteoblast cellular expression, giving rise to better cellular differentiation and better osteoblast expression. The consequence of this effect is an improved osseointegration and a more bone formation.

Also, some more research-based manufacturers, such as Nobel Biocare, have designed such surface treatments so that they increase the thickness and the crystallinity of the titanium oxide layer, as some studies seem to suggest a relationship between the degree of crystallinity and better osseointegration of the implant (Sul et al., "Oxidized implants and their influence on the bone response", J Mater Sci: Mater in Medicine (2002); 12:1025-1031).

The methods used in the state of the art to increase surface roughness of an implant are very diverse. Among them can be highlighted the application of a coating over the surface, blasting of the surface with particles and chemical attack of the surface.

The common methods of coating the metal implant surface consist in applying a metal coating, normally of titanium, or a ceramic layer, normally of hydroxyapatite, by various known techniques such as plasma pulverisation or plasma spray (Palka, V. et al., "The effect of biological environment on the surface of titanium and plasma-sprayed layer of hydroxyapatite". Journal of Materials Science: Materials in Medicine (1998) 9, 369-373).

In the case of blasting the surface, particles of various materials and sizes are used, which are blasted on the surface of the implant in such as way as to alter its morphology. Usually, particles of corundum (alumina) are used (Buser et al. 1991, supra; Wennerberg et al. 1996; supra), or particles of titanium oxide (Gotfredsen, K. et al., Anchorage of TiO2-blasted, "HA-coated, and machined implants: an experimental study with rabbits"., J Biomed Mater Res (1995) 29, 1223-1231).

On the other hand, chemical attack of the surface is carried out using various mineral acids such as hydrofluoric acid, hydrochloric acid, sulfuric acid, etc. So, for example, in a series of United States patents by Implant Innovations Inc. (US 5,603,338; US 5,876,453; US 5,863,201 and US 6,652,765), a two-stage acid treatment is described that is used to obtain the commercial Osseotite® surface. In the first stage, aqueous hydrofluoric acid is used to remove the natural oxide layer on the metal surface; in the second stage a mixture of hydrochloric acid and sulfuric acid is used to obtain a micrometric rough surface. In the European patent application EP 1477 141, also from Implant Innovations Inc., a variation of this method is described in which a mixture of hydrofluoric acid and hydrochloric acid is used in the second stage to treat implant surfaces based on titanium and Ti6Al4V alloys.

The combined use of both techniques has also been described, that is, blasting of the implant surface followed by chemical attack. So, Buser (Buser et al. 1991, Buser at al. 1998, supra) described, among other methods, blasting with medium grain alumina followed by etching with a mixture of hydrofluoric and nitric acids; also blasting with coarse alumina followed by chemical treatment with a mixture of hydrochloric and sulfuric acids. Similarly, Cochran (Cochran et al. 1994, supra) used blasting with fine or coarse corundum particles followed by a chemical treatment with hydrochloric and sulfuric acids to treat a titanium surface. Similarly, Choi Seok et al. (KR 2003007840) described blasting with calcium phosphate particles followed by treatment with a mixture of hydrochloric and sulfuric acids. Equally, in the document WO 2004/008983 by Astra Tech, a method of treatment of implant surfaces was described that combined blasting with fine and coarse particles of titanium oxide followed by treatment with hydrofluoric acid. Also, Franchi (Franchi et al., (2004) "Early detachment of titanium particles from various different surfaces of endosseous dental implants", Biomaterials 25, 2239-2246) and Guizzardi (Guizzardi et al., (2004) "Different titanium surface treatment influences human mandibular osteoblast response", J Periodontol 75, 273-282) described blasting with fine and coarse zirconia particles followed by an unspecified acid treatment.

Regarding thermal treatment, Browne (Browne et al. (1996), "Characterization of titanium alloy implant surfaces with improved dissolution resistance", Journal of Materials Science: Materials in Medicine 7, 323-329) and Lee (Lee et al. (1998), "Surface characteristics of Ti6Al4V alloy: effect of materials, passivation and autoclaving", Journal of Materials Science: Materials in Medicine 9, 439-448) described the treatment of a previously untreated titanium alloy with hot air at 400ºC for 45 minutes to achieve better resistance to dissolution and a higher thickness of the oxide layer; although the thickness achieved was only 4nm.

By means of these methods, therefore, surfaces with micrometric roughness are obtained but with a very much reduced surface titanium oxide thickness, which entail the disadvantages of not having a very stable titanium oxide layer and not reducing the release of metal ions to the medium.

The state of the art, therefore, continues to require alternative methods of treating the surface layer of metal implants that provide a micrometric surface roughness and with improved chemical composition and thickness in order to optimise the process of their osseointegration.

The present inventors have been surprised to find that blasting zirconium oxide particles under pressure on the external area of implants, together with a particular combination of acids for subsequent chemical treatment and a suitable final thermal treatment enables obtaining surfaces with micrometric roughness, a composition that is practically free from impurities and a thickness that is approximately three times the thickness of conventional surfaces. This surface also exhibits good porosity and a morphology that is similar to that of trabecular or spongy bone; characteristics that are very advantageous for optimising the osseointegration and bone anchor processes.

The use of a mixture of sulfuric and hydrofluoric acids, as well as the combination of these three treatments has not been previously described. The selection of suitable thermal treatment of surfaces that have been blasted and subsequently treated with acids has also not been described.

The method of the present invention, which combines these technical characteristics, therefore enables obtaining surfaces of titanium-based metal implants with very good osseointegration properties and cellular response. Additionally, the very significant increase in the thickness of the surface layer of titanium oxide results in great benefits as regards the reduction in metal ion release, an almost stoichiometric titanium oxide composition and its higher wettability of this surface.

### OBJECT OF THE INVENTION

The object of the present invention, therefore is to provide a surface of a titanium-based metal implant to be inserted into bone tissue that comprises substantially pure titanium oxide and has a thickness of 8-50nm.

Another object of the invention is to provide a titanium-based metal implant to be inserted into bone tissue that exhibits this surface.

Another object of the invention is to provide a procedure for obtaining said surface.

### DESCRIPTION OF THE FIGURES

- Figure 1: shows a micrograph (150x) of the surface of the invention.
- Figure 2a: shows the roughness in three dimensions of the surface according to the invention, obtained by confocal microscopy.
- Figure 2b: shows the reconstruction of the flat zone at the end of the implant and the measurement of the corresponding roughness.
- Figure 3: shows the energy dispersive X-ray spectrum (EDS) of the surface of the invention.
- Figure 4: shows the cell viability at 12, 24 and 72 hours as an indicator of the cytotoxicity of the surface of the invention (03/136/15) with respect to other conventional surfaces.
- Figure 5: shows the alkaline phosphatase activity at 6 days of culture as an indicator of the production of bone matrix by osteoblasts seeded on the surface of the invention (03/136/15) with respect to other conventional surfaces.

### DETAILED DESCRIPTION OF THE INVENTION

The present invention provides a surface of a titanium-based metal implant to be inserted into bone tissue comprising substantially pure titanium oxide and having a thickness of 8-50nm, hereinafter called "the surface of the invention".

As previously described, the surface of a titanium-based implant is the superficial layer or its most external zone, composed mainly of titanium oxide.

In a particular embodiment, the surface of the invention has a thickness of 10-30nm. In a preferred embodiment, the surface of the invention has a thickness of 15nm.

This thickness, which is almost three times the thickness of conventional surfaces, means a better osseointegration of the implant as well as an important reduction of impurities, as was previously described.

So, in the context of the invention, the term "substantially pure titanium oxide" refers to the fact that the surface of the invention comprises an almost stoichiometric titanium oxide composition of approximately 98% by weight. (Percentage measured by XPS or X-ray photoelectron spectroscopy, after 1 minute of sputtering, or bombardment with accelerated ions, to eliminate contamination present in the external area of the obtained surface, which is inherent to the analysis method, and obtaining its real composition).

In addition, the surface of the invention exhibits a good micrometric roughness and, therefore, very good osseointegration and cellular response properties. In fact, the morphology of the treated surface is similar to trabecular bone, optimal for the start of bone repair. Also, its characteristics of porosity and roughness enable the homogenisation of residual tensions, adhesion and fixing of initial proteins and the adhesion, proliferation and cellular maturation and stability of the extracellular matrix.

In another aspect of the invention, therefore, a titanium-based metal implant, which exhibits the surface of the invention previously described, is provided, to be inserted into bone tissue.

In a particular embodiment, this metal implant is an implant of titanium or an alloy of titanium. The titanium can be, for example, commercially pure titanium. Equally, the titanium alloy can be any alloy of titanium such as the titanium, aluminium and vanadium Ti6Al4V alloy.

This titanium-based metal implant is appropriate to be inserted into bone tissue, so it can be a dental, orthopaedic, etc. implant, depending on the bone tissue into which it is intended to be inserted.

So, in a particular embodiment, this metal implant is a dental implant.
In another aspect of the invention, a procedure is provided for obtaining a surface of a titanium-based metal implant to be inserted into bone tissue, hereinafter called the "procedure of the invention", which comprises the following stages:
(a) blasting particles of zirconium oxide under pressure onto the external area of the implant;
(b) chemically treating the blasted external area of the implant with an acid composition comprising sulfuric and hydrofluoric acids; and
(c) thermally treating the blasted and chemically treated external area of the implant by heating at a temperature of 200-450ºC for 15-120min.

In another particular embodiment of this procedure, the blasting of particles of zirconium oxide on the external area of the implant in stage (a) is carried out at a pressure of 2-10atm. In a preferred embodiment, the blasting of the particles of zirconium oxide is carried out at a pressure of 6atm.

In another particular embodiment of the procedure of the invention, the particles of zirconium oxide used in stage (a) have a particle size of 25-500pm. In a preferred embodiment, these particles of zirconium oxide have a particle size of 125µm.

After blasting with particles of zirconium oxide, there are no biocompatibility problems if any debris of them remains at the end of the process as this is a very biocompatible material. Also, particles of this material and size are relatively round, which, combined with the working pressure of blasting, produces a concave impact point, ideal for the good cellular response.

To carry out the blasting, or homogenisation of machining stresses, with particles of zirconium oxide, any suitable equipment can be used, such as the Basic Quattro blasting equipment model from Renfert. This equipment is connected to a pressurised air circuit, which blasts the zirconia beads that are loaded into the machine. After the blasting, the surface is cleaned by any suitable method, such as applying compressed air followed by ultrasound cleaning treatment.

In a particular embodiment of the procedure of the invention, the acid composition used in stage (b) comprises 15-50% (v/v) of sulfuric acid and 0.01-1% (v/v) of hydrofluoric acid. In a preferred embodiment, this acid composition comprises 28.8% (v/v) of sulfuric acid and 0.024% (v/v) of hydrofluoric acid.

This particular combination of acids produces a particular roughness and morphology, which, combined with the surface chemical composition obtained with scarcely any impurities, produces an optimum cellular response.

In another particular embodiment of the procedure of the invention, the chemical treatment in stage (b) is carried out at a temperature of 50-110ºC for 4-60min. In a preferred embodiment, this chemical treatment is carried out at a temperature of 75ºC for 12min.

For the chemical attack treatment, standard laboratory equipment is used inside a fume hood (for example, Cruma model 9001-GH air fume hood) to protect against acid vapours. After the chemical treatment, the implant is removed from the acid bath, washed to remove remaining acid and then cleaned by ultrasound and dried. For drying, a conventional drying apparatus can be used, such as the Renfert drying oven.

In another particular embodiment of the procedure of the invention, the thermal treatment in stage (c) is carried out at a temperature of 285ºC for 60min.

This thermal treatment at the indicated temperature and for the stipulated duration causes a re-structuring of the surface titanium oxide layer with an increase in crystallinity and a reduction in impurities, which results in a better cellular response. In addition, this thermal treatment increases the thickness of the surface titanium oxide layer. Under normal conditions, titanium exposed to the atmosphere oxidises and forms a titanium oxide layer some 5 nanometres in thickness. This oxide layer protects the rest of the titanium from oxidising. So, it would be useful to obtain a titanium oxide layer of greater thickness, but not so great that the fragility of the layer could produce micro-particles when the implant rubs against the bone during insertion. In this sense, the range of thicknesses obtained by the procedure of the invention of 8 to 50nm is acceptable.

So, to obtain the desired thickness, thermal treatment must be carried out at a sufficiently high temperature to accelerate the diffusion of atmospheric oxygen into the material, but not so high as to cause oxidation of the titanium that will be visible by a change in colour. To achieve this, the selected working temperature varies between 200ºC and 450ºC.

Finally, the treatment time is the second parameter that must be controlled. A short time does not allow effective diffusion of oxygen. A time that is too long causes excessive increase in the thickness of the layer and makes it unworkable. A reasonable range between these two extremes would be between 15 minutes and 24 hours, depending on the treatment temperature. Therefore, the selected working time varies between 15 and 120 minutes.

The thermal treatment is carried out using conventional methods, for example using a Memmert model UM-100 low-temperature oven.

Another aspect of the invention provides a surface obtainable by the procedure of the invention previously described. This surface comprises substantially pure titanium oxide and has a thickness of 8-50nm, as indicated above. In a particular embodiment, this surface has a thickness of 10-30nm. In a preferred embodiment, this surface has a thickness of 15nm.

In another aspect of the invention, a titanium-based metal implant, which exhibits the surface of the invention previously described, is provided, to be inserted into bone tissue. In a particular embodiment, this metal implant is an implant of titanium or an alloy of titanium. In another particular embodiment, this metal implant is a dental implant.

The following examples illustrate the invention and must not be considered as limiting its scope.

### EXAMPLE 1

### Obtaining a titanium dental implant with a surface according to the invention.

A commercially pure titanium threaded conical-cylindrical endosseous Defcon TSA implant was subjected to blasting with zirconia particles of 125µm at a pressure of 6atm, placing the exit orifice perpendicular to the surface being treated at a distance of between 2cm and 3cm. After blasting, it was cleaned with pressurised air and then submerged in pure water in ultrasound for 10 minutes. It was then dried using compressed air.

An aqueous solution was then prepared with the following composition: 28.8% by volume of sulfuric acid and 0.024% by volume of hydrofluoric acid. The beaker with the reagents was placed in a thermal bath, setting the temperature at 75º+/-2ºC. Once the desired temperature had been reached by the reagent, the blasted implant was subjected to chemical treatment by immersing it in the reagent for 12 minutes (+/-15 seconds). At the end of this treatment, the implant was removed from the acid bath and then washed by shaking for 15 seconds in two consecutive baths of pure water. Then, it was submerged in pure water in ultrasound for 10 minutes and then dried in an oven.

Finally, the treated implant was subjected to a final thermal treatment at a temperature of 285ºC (+/-20ºC) for 60 minutes in a Memmert model UM-100 low-temperature oven.

### EXAMPLE 2

### Characterisation of the surface obtained in example 1

### Morphology

The morphology of the surface obtained in example 1 was examined by surface micrographs and measurement of the roughness by confocal microscopy.

### Surface micrographs

Micrographs of the surface were carried out in a JEOL JSM 840 scanning electronic microscope with a 15kV scanning beam.

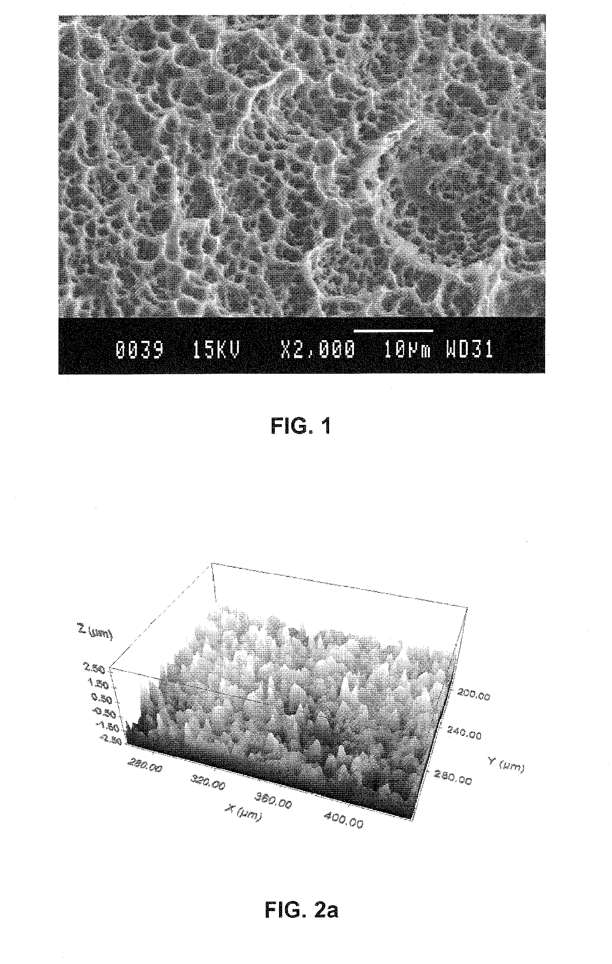

Figure 1 shows a micrograph (150x) of this surface, where it can be seen that the surface exhibits a very characteristic superficial roughness, with roughness value Ra (average roughness) of around 1 µm, characterised by rounded morphology with sharp external edges and the presence of deep porosity distributed homogenously, due to the action of the attack acid over the surface.

This level of roughness complies with requirements indicated in various scientific articles (Buser et al. 1991, Cochran et al. 1994, Martin et al. 1995, Wennerberg et al. 1996, Wennerberg et al. 1997, Buser et al. 1998, Lazzara et al. 2000, Orsini et al. 2000, supra) with respect to the need for the implant surface to have a roughness that enables good anchoring of the cells.

### Measurement of the roughness by confocal microscopy

The measurement of roughness in 3D was carried out by a confocal microscope connected to PLµ software developed by the Department of Optics of the Escuela Técnica Universitaria de Terrassa (Polytechnic University of Catalonia). The measurements were made according to the DIN 4768 standard with a Gaussian cut-off filter of 800µm.

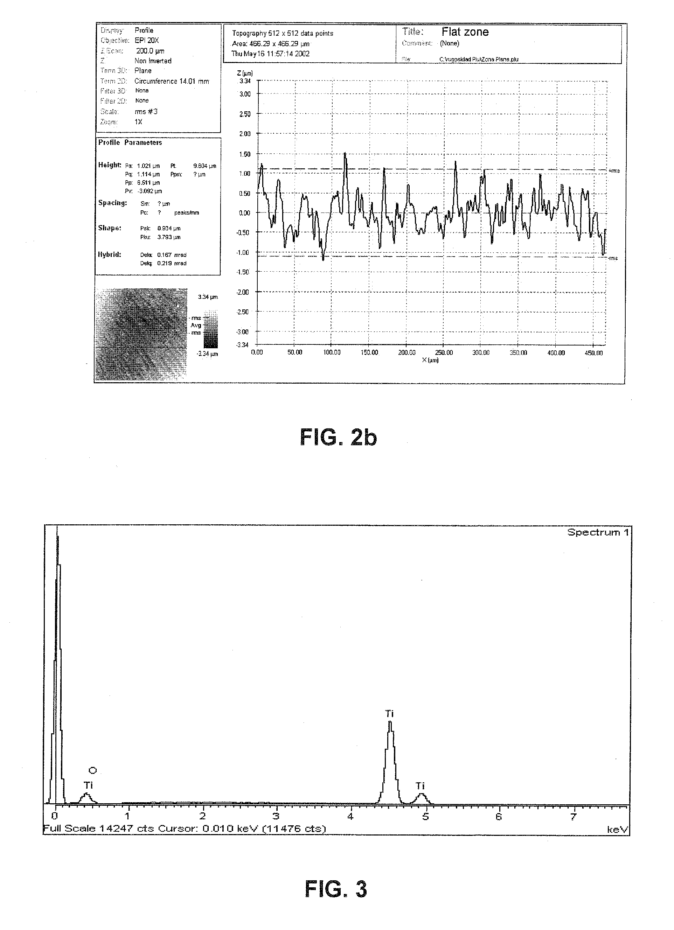

Figure 2a shows the roughness in three dimensions of the surface obtained by this technique. Also, figure 2b shows the reconstruction of the flat zone at the end of the implant and the measurement of the corresponding roughness according to a transverse profile of the surface.

The values of roughness obtained gave average values of Ra (average roughness) of 1.0µm, with spacing between peaks Sm of 12µm. These values are close to the values cited as desirable in the bibliography referred to in the previous section.

### Surface chemical composition

The analysis of the surface chemical composition was carried out by two different techniques: energy dispersive X-Ray spectroscopy (EDS) and X-ray photoelectron spectroscopy (XPS).

### Energy dispersive X-Ray spectroscopy (EDS) analysis

This technique enables the determination of the quantitative composition of a surface in a thickness of approximately 1µm with a high spatial resolution. EDS enables the detection of the presence of atoms with an atomic weight between that of boron and uranium and the quantification of that presence on the surface under investigation.

EDS measurements were carried out by Scientific-Technical Services of Barcelona University. A Leica Electroscan 360 SEM was used with Link-Inca EDS equipment capable of detecting atoms with an atomic weight equal to or greater than that of boron. Figure 3 shows the energy dispersive X-ray spectrum (EDS) obtained.

The analysis carried out by EDS demonstrated the presence of only titanium and oxygen on the surface of the treated sample of titanium, with a small trace of zirconium. The presence of zirconium is due to the previous homogenisation of machining stresses treatment that can leave some particles of zirconium oxide sticking to the surface. Various analyses showed that this behaviour is observed across the whole surface of the treated implant.

### X-Ray Photoelectron spectroscopy (XPS) analysis

XPS analyses were carried out by the ESCA and TEM Unit of Scientific-Technical Services of Barcelona University. The results are shown in Table 1, together with a comparison with XPS analysis cited in the bibliography of various dental implants (Wieland et al., "Measurement and evaluation of the chemical composition and topography of titanium implant surfaces", Bone Engineering, ch.14 (2000) J.E.Davies eds; Massaro et al., "Comparative investigation of the surface properties of commercial titanium dental implants. Part I: chemical composition". J Mat Sci: Mat in Medicine (2002) 13: 536-548).

**Table 1. Results of the analysis of the surface of samples by XPS, comparing with those of the surfaces of other commercial implants.**

| | **C (%)** | **O (%)** | **Si (%)** | **N (%)** | **Ti (%)** | **Na (%)** | **Cl (%)** | **TiO₂ layer (nm)** |
|---|---|---|---|---|---|---|---|---|
| **Surface¹** | 27.7 | 51.2 | - | 0.6 | 19.8 | - | - | 15 |
| **"Sputtering" 1 minute²** | 1.8 | 59.8 | - | - | 36.4 | - | - | 15 |
| **Machined Branemark³** | 29.8 | 51.9 | - | - | 12.8 | 5.0 | 0.5 | 5.7 |
| **ITI SLA⁴** | 34.9 | 51.4 | traces | 1.3 | 14.5 | - | - | 5.7 |
| **3i Osseotite⁵** | 53.7 | 36.2 | 3,3 | 5.4 | 6.8 | traces | traces | N.a. |

| | | | | | | | | |
|---|---|---|---|---|---|---|---|---|
| 1- Surface of the invention analysed without "sputtering" (includes the detection of contamination present in the most external area of the surface obtained and that is inherent to the analysis method). 2- Surface of the invention after 1 minute of "sputtering" 3- Machined Brånemark: untreated, only mechanical process (Nobel Biocare). 4- ITI SLA: blasting + acid treatment (Straumann). 5- 3i Osseotite: acid treatment (Biomet 3i). N.a.: Not available | | | | | | | | |

Comparison of the results shows that the chemical composition of the surface of the invention is comparable to that of other implants available on the market, with even less carbon and silicon contamination (Wennerberg et al. 1996, supra; Wieland et al. 2000, supra; and Sittig et al., "Surface characterization of implant materials c.p. Ti, Ti6Al7Nb and Ti6Al4V with different pretreatments", J Mater Sci: Mater in Medicine (1999), 10:35-46).

The presence of some elements on the surface, such as nitrogen, is due to the thermal treatment process. Other contaminants common in other processes, such as silicon and sodium, were not detected. The residual % to make up 100% is due to the argon detected (not indicated), which is a residue of the process of measurement with XPS.

### EXAMPLE 3

### Cellular response of the surface of a sample of titanium obtained by a process analogous to that described in Example 1.

A study was carried out by the 063-13 research group (Pharmacology Dept. Faculty of Medicine and Odontology, Santiago de Compostela University, Spain) on the biological evaluation of titanium samples (commercially pure titanium discs of 5mm diameter) treated by a process analogous to that described in Example 1.

Human osteoblasts were seeded on working samples (8x10³ cells/disc in triplicate) in modified Dulbecco culture medium with 10% foetal calf serum and 1% antibiotic solution. The cellular bioactivity (indicator of the cytotoxicity of the surface) and the production of alkaline phosphatase (indicator of the production of bone matrix by osteoblasts) were measured for the surface of the invention (code 03/136/15) compared to those of an untreated surface of the same titanium subjected to machining (code 03/137/07), another untreated surface of the same titanium subjected to blasting (polishing with 5 micrometre silicon carbide paper) (code 03/136/18) and an untreated surface subjected to blasting plus acid treatment similar to the ITI SLA surface (code 03/136/09).

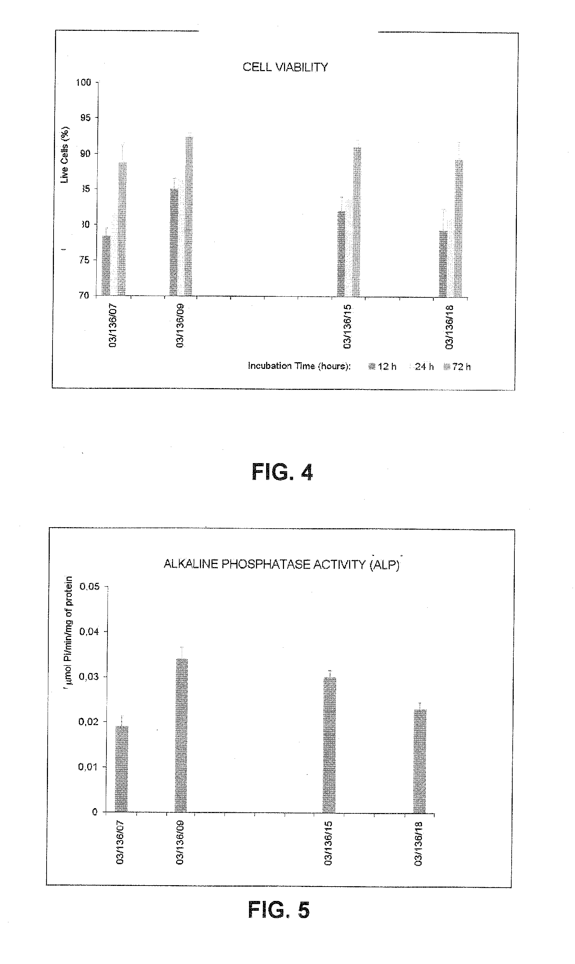

Figure 4 shows the results of the cellular viability measurement at 12, 24 and 72 hours in these samples. Figure 5 shows the results of the alkaline phosphatase activity measurements after 6 days of culture of these samples.

Alkaline phosphatase activity has been for a long time associated with biological calcification. Thus, improved expression of this enzyme seems to be necessary before the start of bone matrix mineralisation, providing the localised enrichment of inorganic phosphate for nucleation and proliferation of hydroxyapatite crystals, a main component of bone tissue.

As can be seen in Figures 4 and 5, the results obtained show a better cellular response by the surface of the invention (code 03/136/15) compared to the surface of the machining (code 03/136/07) and blasting (code 03/136/18) controls. The results of the cellular response of the surface of the invention are similar to those of the blasted and acid treated control (code 03/136/09).

## Claims

1. Surface of a titanium-based metal implant to be inserted into bone tissue, **characterised in that** it comprises substantially pure titanium oxide and has a thickness of 8-50nm.

2. Surface according to claim 1, **characterised in that** it has a thickness of 10-30nm.

3. Surface according to claim 2, **characterised in that** it has a thickness of 15nm.

4. Titanium-based metal implant to be inserted into bone tissue **characterised in that** it exhibits the surface defined in claims 1-3.

5. Implant according to claim 4, **characterised in that** it is an implant of titanium or an alloy of titanium.

6. Implant according to claim 4, **characterised in that** it is a dental implant.

7. Procedure for obtaining a surface of a titanium-based metal implant to be inserted into bone tissue **characterised in that** it comprises the stages of:
(a) blasting particles of zirconium oxide under pressure onto the external area of the implant;
(b) chemically treating the blasted external area of the implant with an acid composition comprising sulfuric and hydrofluoric acids; and
(c) thermally treating the blasted and chemically treated external area of the implant by heating at a temperature of 200-450ºC for 15-120min.

8. Procedure according to claim 7, **characterised in that** the blasting of zirconium oxide particles on the surface in stage (a) is carried out at a pressure of 2-10atm.

9. Procedure according to claim 8, **characterised in that** the blasting of zirconium oxide particles on the surface in stage (a) is carried out at a pressure of 6atm.

10. Procedure according to claim 7, **characterised in that** the particles of zirconium oxide used in stage (a) have a particle size of 25-500µm.

11. Procedure according to claim 10, **characterised in that** the particles of zirconium oxide used in stage (a) have a particle size of 125µm.

12. Procedure according to claim 7, **characterised in that** the acid composition used in stage (b) comprises 15-50% (v/v) of sulfuric acid and 0.01-1% (v/v) hydrofluoric acid.

13. Procedure according to claim 12, **characterised in that** the acid composition used in stage (b) comprises 28.8% (v/v) of sulfuric acid and 0.024% (v/v) hydrofluoric acid.

14. Procedure according to claim 7, **characterised in that** the chemical treatment in stage (b) is carried out at a temperature of 50-110ºC for 4-60min.

15. Procedure according to claim 14, **characterised in that** the chemical treatment in stage (b) is carried out at a temperature of 75ºC for 12 min.

16. Procedure according to claim 7, **characterised in that** the thermal treatment in stage (c) is carried out at a temperature of 285ºC for 60 min.

17. Surface obtainable by the procedure of claims 7-16, **characterised in that** it comprises substantially pure titanium oxide and **in that** it has a thickness of 8-50nm.

18. Surface according to claim 17, **characterised in that** it has a thickness of 10-30nm.

19. Surface according to claim 18, **characterised in that** it has a thickness of 15nm.

20. Titanium-based metal implant to be inserted into bone tissue **characterised in that** it exhibits the surface defined in claims 17-19.

21. Metal implant according to claim 20, **characterised in that** it is an implant of titanium or an alloy of titanium.

22. Metal implant according to claim 20, **characterised in that** it is a dental implant.
